# EUROPEAN PATENT APPLICATION

(11) **EP 4 691 381 A1**
(43) Date of publication of application: **11.02.2026**
(21) Application number: 25193812.2
(22) Date of filing: 04.08.2025
(51) Int. Cl.: A61B 17/14, B22F 5/10, B22F 7/08

(54) **ADDITIVE MANUFACTURING PART SUPPORT AND HANDLING FRAME**

(30) Priority: 07.08.2024 US 202463680141 P
(71) Applicant: Howmedica Osteonics Corp., Mahwah, NJ 07430 (US)
(72) Inventor: Kenny, Mark, Loughrea, Co. Galway (IE)
(74) Representative: Keller Schneider Patentanwaltsgesellschaft mbH

(57) **Abstract**

A cutting instrument including an insert and a frame having sidewalls that extend from a base and define a slot. The slot is configured to receive a portion of the insert such that an edge of the insert extends beyond the frame. The sidewalls are configured to shrink around a portion of the insert such that an edge of the insert extends beyond the frame. Additionally, a method for fabricating a cutting instrument including depositing layers of metal powder onto a base and selectively binding the deposited layers of the metal powder onto the base to form a frame attached to the base. Next, placing an insert within the frame such that a portion of the insert extends beyond the frame and the combination of the base, the frame, and the insert forms an in-process assembly.

## Description

### BACKGROUND

Cutting instruments are heavily relied upon in the medical field. Such instruments require sharp and durable cutting blades that allow for efficient and precise cutting of soft tissue and/or bone during surgical procedures. When a blade of a surgical instrument becomes dull, the cutting edge of the blade may be sharpened, or the blade may be altogether replaced to restore the cutting capabilities of the instrument. While the use of replacement blades for cutting instruments may be more convenient than sharpening, manufacturing high-quality replacement blades suited for surgical procedures can be costly.

Accordingly, there remains a need for designs and methods that reduce the costs of manufacturing replacement blades for surgical instruments without sacrificing the quality or capability of the blades.

### BRIEF SUMMARY

In some arrangements, this disclosure relates to a cutting instrument that utilizes a blade having pockets that are each configured to receive at least one cutting insert. The cutting insert is inserted into a pocket when the pocket is in a green state (i.e., a state prior to heat treatment such as sintering) to form an in-process (i.e., unfinished) assembly intended to undergo further manufacturing processing before achieving a finished state. For example, the pocket may be formed through a binder jetting process where layers of powder are selectively bound together with a binding agent, but the layers have not been heat treated and thus are in a green state. After the cutting insert is aligned and positioned within the pocket, the cutting insert is permanently fixed to the blade after the pocket is heat treated. During heat treatment, the walls of the pocket shrink around a portion of the cutting insert such that a cutting edge of the cutting insert extends beyond the walls of the pocket and away from the instrument. In this manner, the cutting insert may have various cutting edges that are exposed. The blade may include multiple cutting inserts that are attached to the blade in this way.

The pockets of the blade may be specifically tailored to accommodate various shapes of cutting inserts. In some arrangement, the cutting inserts may have a lower engagement portion that fits within a pocket and cutting portion that extends beyond the pocket and defines complex cutting surfaces similar to the cutting surfaces disclosed in PCT Pub. No. WO2022133340A2, the disclosure of which is incorporated herein by reference. The cutting inserts may be designed such that each of the cutting portions define different arrangements of cutting surfaces, but the engagement portions of the cutting inserts may be all the same shape and dimensions that correspond a certain pocket design that is common among several different types of blades. In this manner, there is versatility and flexibility in the design and arrangement of the cutting surfaces for various blades.

In accordance with other arrangements, a cutting instrument may include an insert and a frame. The insert may be disposed within the frame. The frame may include sidewalls that extend from a base of the frame and may define a slot. The slot may be configured to receive the insert such that an edge of the insert extends beyond the frame and the sidewalls are configured to shrink around a portion of the insert. The frame may be made of metal powder and a binding agent binding the metal powder. The insert may include a cutting edge configured to cut through bone tissue.

In other aspects, the cutting instrument may include an additional frame extending from the base and an additional insert. The additional frame may be configured to receive the additional insert. The insert may include a first tip that may be part of the edge of the insert. The additional insert may include a second tip. The frame and the additional frame may be adjacent to each other and oriented such that the first and the second tips point in the same direction and are spaced an equal distance from the base when the insert and the additional insert are inserted into the frame and the additional frame, respectively. A width of the frame may decrease as the frame extends away from the base. The frame may further include a backwall that extends between the sidewalls. The frame may be integral with the base such that the base and the frame are inseparable. The insert may be formed by subtractive manufacturing methods. The frame may be formed by additive manufacturing methods. The insert may be made of a single material. The insert and the frame may be made of different materials. The insert and the additional insert may have different shapes. The sidewalls may be configured to hold the insert in a fixed position after the sidewalls are heated treated.

According to another aspect or arrangement, a cutting instrument may be fabricated by a process. In this process, layers of a metal powder may be deposited onto a base. Next, the deposited layers of the metal powder may be selectively bound onto the base to form a frame attached to the base. An insert may be placed within the frame such that a portion of the insert extends beyond the frame when the frame is in a green state. The insert and the frame may form an in-process assembly. Additionally, prior to forming the frame, layers of the metal powder may be deposited onto a substrate, and the deposited layers of the metal powder may be selectively bound onto the substrate to form the substrate. Prior to forming the frame, layers of the metal powder may be deposited onto a substrate and then the deposited layers of metal powder may be selectively bound onto the substrate to form the substrate. The process may further include a heat treatment that is applied to the in-process assembly to cause the frame to shrink around a portion of a perimeter of the insert. During the heat treatment, the frame may shrink by 15-20% (i.e., 15-20% of its original size). Such shrinkage of the frame may not be uniform (i.e., non-uniform shrinkage). The depositing step may further include depositing layers onto the base to form an additional frame that is attached to the base and adjacent to the frame. The placing step may include placing an additional insert into the additional frame. The process may further comprise fabricating the insert by a machining process before the placing step. The process may further comprise separating the frame from the base by detaching the deposited layers from the base. The process may further include layers of metal powder that are deposited onto the base, and the deposited layers may be selectively bound onto the base to form a replacement frame attached to the base. Then a replacement insert may be placed within a replacement frame such that a portion of the replacement insert extends beyond the replacement frame. The combination of the base, the replacement frame, and the replacement insert forms an in-process assembly.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIG. 1 is a perspective view of a cutting tool that has a blade disposed within a blade bar operably attached to the cutting tool in accordance with an aspect of the present disclosure;
FIG. 2A is an exploded view the blade bar and the blade of FIG. 1;
FIG. 2B is a focused view of the blade of FIG. 2A;
FIG. 3 is a schematic view of cutting inserts of the blade of FIG. 2B;
FIG. 4A is a schematic view of the blade of FIG. 3 prior to being heat treated;
FIG. 4B is a schematic view of the blade of FIG. 4A without any cutting inserts;
FIG. 5 is a perspective view of a blade having a pocket configured to receive a cutting insert in accordance with an aspect of the present disclosure;
FIG. 6 is a schematic view of cutting inserts attached to a blade in accordance with an aspect of the present disclosure;
FIG. 7A is a schematic view of a blade in a green state receiving a cutting insert in accordance with an aspect of the present disclosure;
FIG. 7B is a schematic view of the blade of FIG. 7A undergoing heat treatment, and
FIG. 8 is a process diagram that shows the steps for forming a blade and attaching cutting inserts to the blade in accordance with an aspect of the present disclosure.

### DETAILED DESCRIPTION

As used herein, the term "distal" means more distant from the heart and the term "proximal" means closest to the heart. The term "inferior" means toward the feet and the term "superior" means towards the head. The term "anterior" means towards the front part of the body or the face and the term "posterior" means towards the back of the body. The term "medial" means toward the midline of the body and the term "lateral" means away from the midline of the body. The term "step of" does not mean "step for." The term "green state" means a state prior to heat treatment (e.g., sintering). For example, in additive manufacturing, a printed part is in a "green state" before the printed part has undergone heat treatment such as furnace sintering. The green state could also include the period during heat treatment but prior to the completion of the heat treatment. The term "in-process assembly" means an assembly of components intended to undergo further manufacturing processing to reach a finished state or its final form.

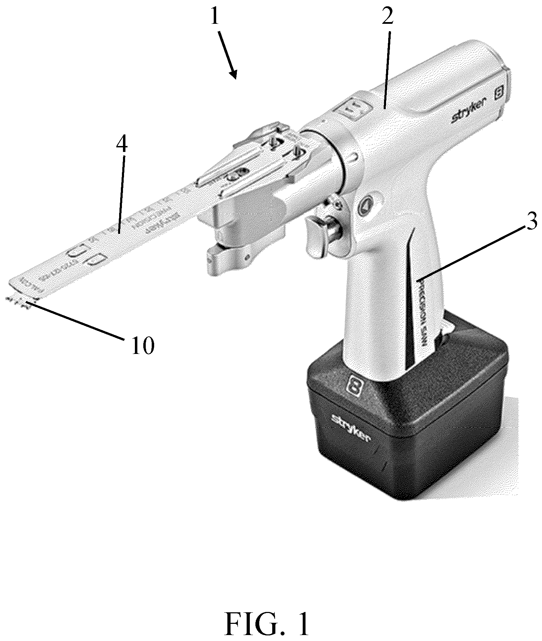

Referring now to the drawings, FIGS. 1-2A illustrates a cutting tool 1 that includes a blade 10 extending from the distal end of a blade bar 4 that is connected to the cutting tool. Cutting tool 1 can be a sagittal saw configured to move blade 10 in an oscillating motion similar to the cutting blades disclosed in PCT Pub. No. WO2022133340A2, PCT Pub. No. WO2006/017066A2, US Pat. No. 7,497,860, PCT Pub. No. WO2007/030793A2, US Pat. No. 7,704,254, PCT Pub. No. WO2016/182981A2, and US Pat. No. 10,687,823, the contents of which are each incorporated herein by reference. For example, cutting tool 1 can have a body portion 2 and a handle portion 3 that extends from the body portion. Body portion 2 is adapted to removably attach to and operatively engage with blade bar 4 and thereby blade 10 such that the cutting tool 1 can move a cutting end 17 of the blade in a cutting motion (e.g., an oscillating motion), the cutting end of the blade extends from the blade bar, as shown in FIG. 1. Blade 10 can have an attachment mechanism 13 that has connection links that are housed within blade bar 4 and configured to operatively connect with head portion 2. Handle portion 3 includes a trigger that is adapted to control the operation of the blade 10, e.g., when needed to cut through bone or soft tissue. When cutting end 17 of blade 10 becomes dull, the blade may be detached and replaced with a new blade of similar configuration. It is contemplated that the various blade embodiments disclosed herein can be used with a variety of cutting instruments other than cutting tool 1, which is not meant to limit the scope of the disclosure in any way.

Such cutting instruments may include various sagittal saws for performing a surgical procedure. For example, a saw of this type may have a static, planar guide bar that extends forward from the saw handpiece. A saw blade is pivotally attached to the distal end section of the guide bar. Drive rods or drive elements are attached to the opposed sides of the blade and are housed within the guide bar. The drive rods are attached to a drive assembly integral with the saw handpiece.

The saw of this disclosure is used by actuating a motor internal to the handpiece. The drive assembly transfers the power developed by the motor to the drive rods so that the rods simultaneously engage in back-and-forth reciprocating motion in opposite directions. The drive rods, in turn, transfer the reciprocating motion to the saw blade so that the blade teeth move in a back-and-forth or side-to-side oscillating motion.

The pivotal attachment of the saw blade at the distal end of the guide bar results in less vibratory motion of the saw. Further, the static guide bar which supports the blade avoids excessive wearing of the cutting guide, and particularly slotted cutting guides. Of particular advantage is the configuration of the guide bar. More specifically, the guide bar is constructed so as to have a relatively small thickness dimension, which in the preferred embodiment is no larger than the thickness dimension of the blade. This allows increased extension or advancement of the blade into the bone, since the guide bar is dimensioned so that same will fit into the cutting slot or kerf created by the blade in the bone. Further, the drive rods are under tension in that same exert a pulling force on the blade to oscillate same. This means that thinner drive rods can be utilized, which results in a lightweight and easy to use saw.

The cutting guide is defined by a block or body. Pins or other fastening members hold the guide block in a fixed position relative to the bone or other hard tissue to be cut. The cutting guide is formed with one or more outer surfaces that are guide surfaces. The cutting guide is positioned so that the guide surface is in the plane or immediately adjacent the plane in which the cut through the bone is to be made. A capture pin extends upwardly from the guide surface. The guide bar of the saw of this disclosure is further formed to have an elongated slot. The cutting guide capture pin is seated in the slot to hold the saw guide bar to the cutting guide, and yet allow the saw to both move forward and pivot relative to the cutting guide. The capture pin can be slidably mounted or seated within an elongate slot or track defined in the block which opens through the guide surface, which capture pin is moved by the guide bar of the saw during a cutting procedure to allow lateral movement of the saw across the guide surface. Alternatively, the capture pin can be mounted to the block in a removable manner to allow same to be mounted in multiple locations along the block.

The capture pin reduces the overall size of the cutting guide, which makes it possible to use a cutting guide that is relatively small in size. Further, the cutting guide according to the disclosure allows greater cutting site visibility of open-face cutting guides as discussed above. However, the capture pin according to the disclosure avoids the disadvantage of conventional open-face cutting guides wherein the surgeon must make a conscious effort to maintain the saw blade flat against the guide surface of the cutting guide.

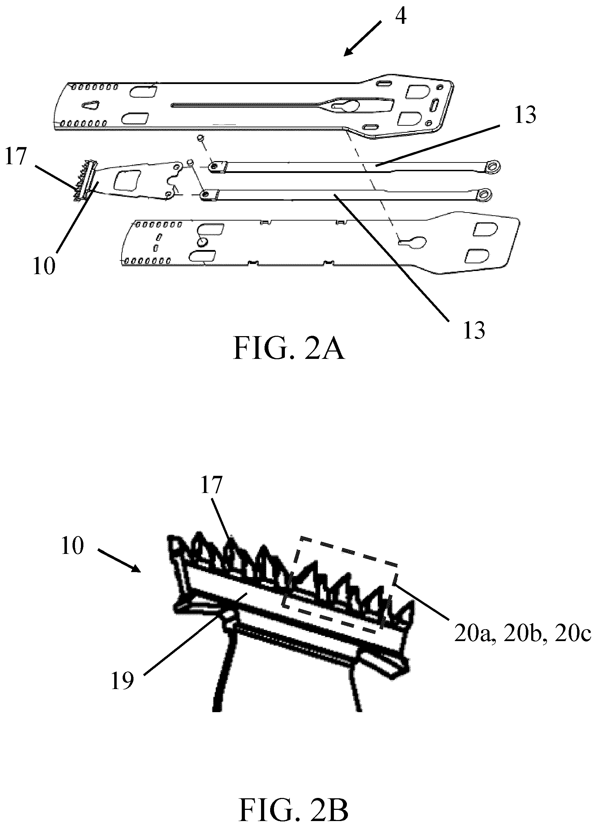

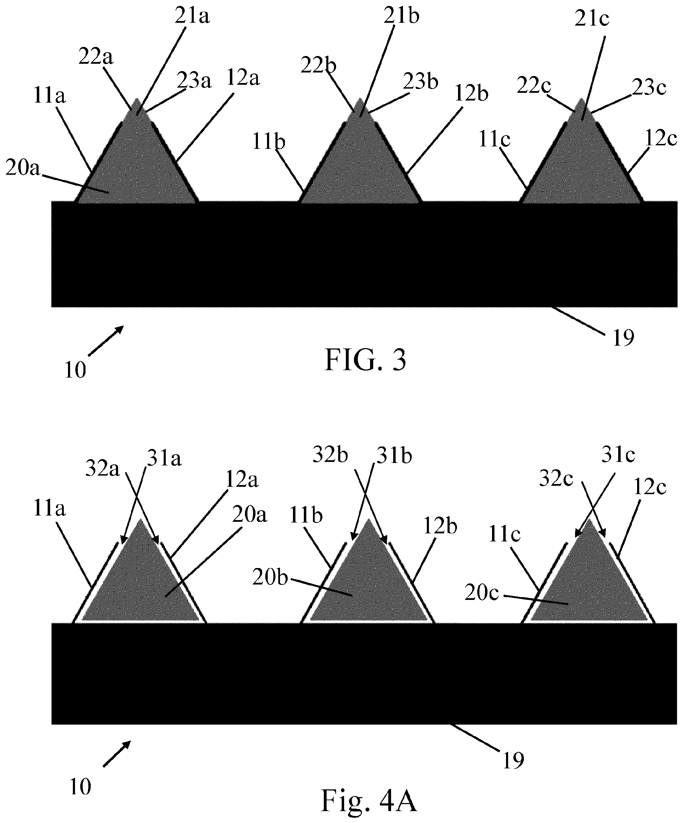

Referring now to FIGS. 2A-6, blade 10 having cutting inserts 20a, 20b, 20c that are disposed at the distal end of the blade and multiple pairs of sidewalls that include a first sidewall 11a and a second sidewall 12a that extend from the distal end of base 19 of the blade toward each other at an oblique angle such that the first and the second sidewalls define a slot 14a having an opening 15a at one end thereof. As shown in FIG. 3, slot 14a narrows as it extends away from blade 10. All the pairs of sidewalls, including first side walls 11a, 11b, 11c and second sidewalls 12a, 12b, 12c, are integral with blade 10.

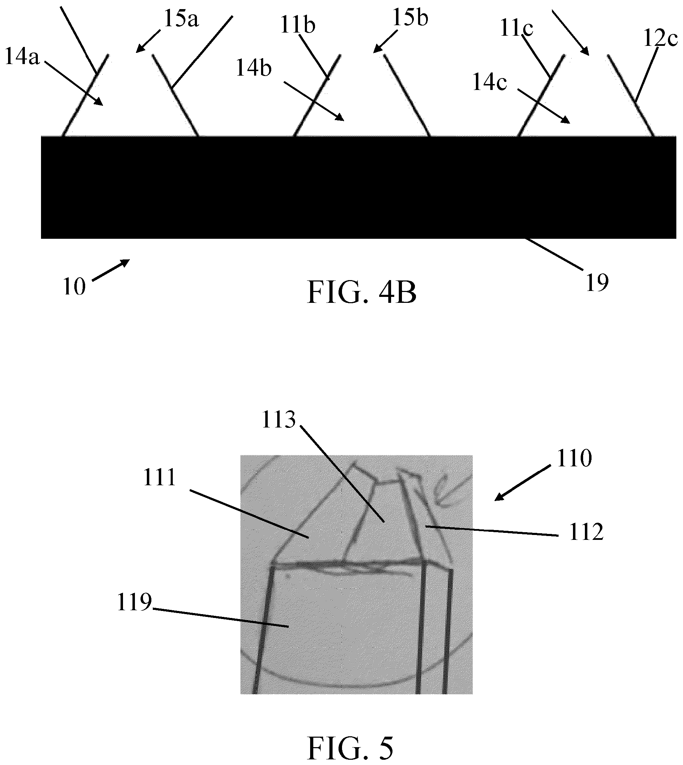

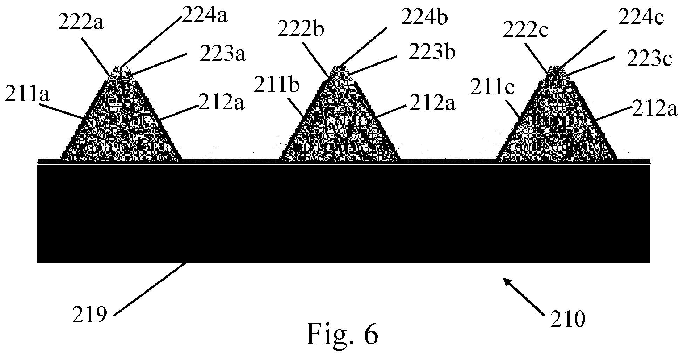

Cutting insert 20a is disposed between first sidewall 11a and second sidewall 12a of the multiple pairs of sidewalls extending from blade 10 such that a tip 21a of the cutting insert extends beyond the sidewalls. In other words, cutting insert 20a is disposed within slot 14a defined by first sidewall 11a and second sidewall 12a such that tip 21a extends beyond the slot, as shown in FIGS. 4A and 4B. Tip 21a defines at least a first cutting edge 22a and a second cutting edge 23a. In some embodiments, cutting insert 20a may include a third cutting edge 24a at the distal end thereof, as shown in FIG. 6. When blade 10 is in a post-process state, i.e., the blade has been heat treated (e.g., sintered), first sidewall 11a and second sidewall 12a are permanently pressed against cutting insert 20a such that the cutting insert is fixed to the distal end of the blade 10, as best shown in FIG. 3. When blade 10 is in an in-process or green state, i.e., prior to the heat treatment, there is a first gap 31a between first sidewall 11a and cutting insert 20a and a second gap 32a between second sidewall 12a and cutting insert 20a, as best shown in FIG. 4A. In other words, when blade 10 is in a green state, cutting insert 20a can be loosely fitted between first sidewall 11a and second sidewall 12a. While the cutting inserts shown in the figures are two-dimensional triangles, the figures are mainly representative as to what is contemplated. In other words, the cutting inserts may have a complex structure that includes various cutting edges extending at different orientations and angles.

In other instances, a blade 110 includes a first sidewall 111, a second sidewall 112 and a back sidewall 113 extending from base 119 of the blade. First sidewall 111 and second sidewall 112 have the same characteristics and features as first sidewall 11a and second sidewall 12a described above. For example, first sidewall 111 and second sidewall 112 define a slot 114 and opening 115 such that both have the same characteristics and features as slot 14a and opening 15a, respectively, as described above. Back sidewall 113 extends from the backside of first sidewall 111 to the backside of second sidewall 112 across slot 114 such that the sidewalls create a pocket 116 that is configured to receive a cutting insert 20, as best shown in FIG. 5. In this manner, cutter insert 20 may be inserted into and loosely held by pocket 116 while the pocket 116 is in a green state. After blade 110 has been heat treated, first side wall 111, second sidewall 112 and back sidewall 113 press against cutting insert 20 in a shrunk state similar to the sidewalls described above. A plurality of blades 110 may be formed together to create a larger blade that has a row of cutting inserts extending along one side of the blade, similar to what is shown in FIGS. 4A and 4B.

The cutting inserts can have various shapes and sizes. For example, as shown in FIG. 6, cutting insert 220 is shaped such that multiple cutting surfaces, namely, cutting surfaces 222a, 223a and 224a extend beyond first and second sides walls 211a, 212a, as opposed to just two cutting surfaces or edges that extend beyond the sidewalls. First sidewall 211a and second sidewall 212a have the same characteristics and features as first sidewall 11a and second sidewall 12a described above.

The cutting inserts described herein may be fabricated by a number of processes, some of which are not discussed herein. In some instances, cutting insert 20 may be formed by die casting and later machined. Such machining may be performed by a computer-aided manufacturing (CAM) process. For example, computer numerical control (CNC) mill may be utilized to machine the cutting inserts. The cutting inserts may be made from various metals and metal alloys including but not limited to steel, stainless steel, carbon steel and the like. The cutter inserts may be designed using a computer-aided design (CAD) software with the design being saved to a digital file that is readable by a CNC machine or an additive manufacturing machine (e.g. a three-dimensional printer). In some instances, the cutter inserts may be fabricated by additive manufacturing methods such as Binder Jetting, Powder Bed Fusion, Direct Energy Deposition, Bound Powder Extrusion or fabrication method similar thereto.

The sidewalls and the blade described herein are fabricated preferably through a binder jetting process that includes a binding phase and a heat treating (e.g., sintering) phase. In some arrangements, the sidewalls and the base of the blade may be designed using a computer-aided design (CAD) software with the design being saved to a digital file. The digital file may be then transferred to a three-dimensional printing machine to fabricate the sidewalls and the base of the blade according to the design on the digital file. The sidewalls and the base of the blade may be made from various metals including but not limited to steel, stainless steel, carbon steel, tool steel, carbide-tipped steel, various metal alloys, and the like. In some instances, the blade and sidewalls thereof are made from a different material than that of the cutting inserts, which allows for cost-effective devices, reduced weight options, and innovative designs.

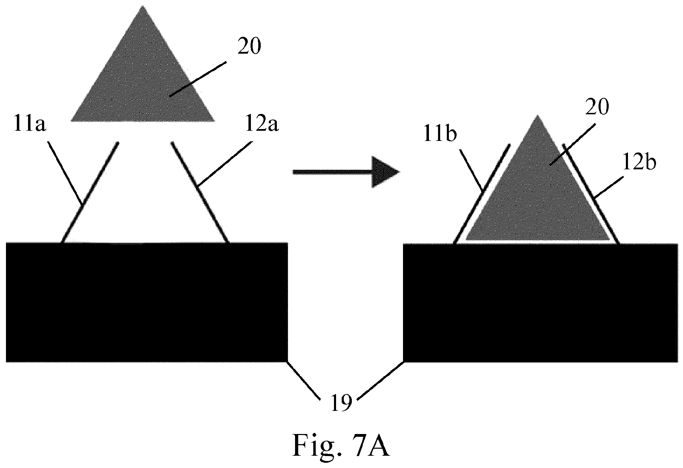

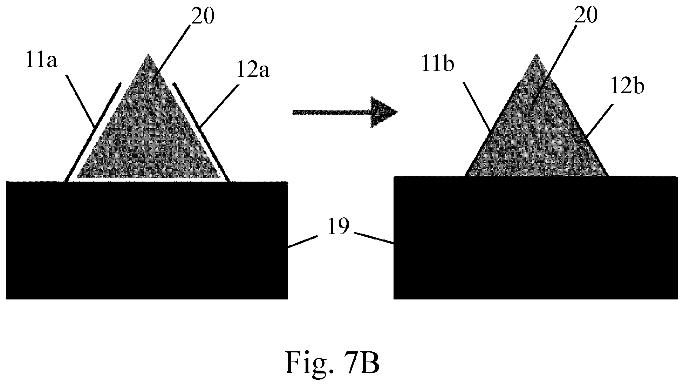

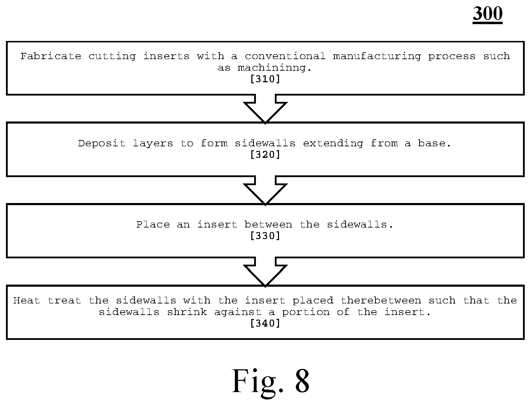

Now referring to FIGS. 7A-8, a blade is fabricated according to process 300. At step 310, a cutting insert, e.g., cutting insert 20a, is fabricated via a machining process as described above. At step 320, layers of metal powder are deposited along with a binding agent to form at least one pair sidewalls, e.g., first sidewall 12a and second sidewall 11a, extending from a base of a blade, e.g., blade 20, to form a green state part. At step 330, the cutting insert is placed within a slot, e.g., slot 14, defined by the pair sidewalls such that the tip of the cutting insert extends out of the slot and beyond the sidewalls, as shown in FIG. 7A, to form an in-process assembly. In some instances, there may be a back sidewall, e.g., back sidewall 113, that extends across the slot along the backsides of the sidewalls to form a pocket, e.g., pocket 116. In such instances, the cutter insert is inserted and placed within the pocket. Additionally, this step may include aligning the cutting insert with the slot before inserting the cutting insert therein. At step 340, the in-process assembly is heat treated, e.g., sintered, which causes the sidewalls to shrink around and press against the cutting insert except for the tip thereof, as shown in FIG. 7B, as a result of debinding. In some instances, during the heat treatment, the sidewalls undergo shrinkage of 15% to 20% along the X, Y, and Z axes. In this manner, the cutter insert is permanently fixed to the blade without further additional assembly steps.

In some arrangement, the shrinkage of the sidewalls may be modeled as an initial step by using a computer software to accurately model the precise shrinkage of the sidewalls. Based on this shrinkage modeling, the dimensions of the cutter insert and the slot formed by the sidewalls of the blade may be determined to ensure that the sidewalls firmly pressed against the cutting insert as a result of heating and debinding phase and thereby securely affixing the cutting insert to the base of the blade.

It is to be understood that the disclosure set forth herein includes any possible combinations of the particular features set forth above, whether specifically disclosed herein or not. For example, where a particular feature is disclosed in the context of a particular aspect, embodiment, arrangement, or configuration, that feature can also be used to the extent possible, in combination with and/or in the context of other particular aspects, embodiments, arrangements, and configurations of the technology, and in the technology in general.

Furthermore, although the technology here has been described with reference to particular features and figures, it is to be understood that these features are merely illustrative of the principles and applications of the present technology. It is therefore to be understood that numerous modifications, including changes in the sizes of the various features described herein, may be made to the illustrative arrangement and that other arrangements may be devised without departing from the spirit and scope of the present technology. In this regard, the present technology encompasses numerous additional features in addition to those specific features set forth in the claims below. Moreover, the foregoing disclosure should be taken by way of illustration rather than by way of limitation as the present technology is defined by the claims set forth below.

## Claims

1. An in-process cutting instrument comprising:
an insert; and
a frame including sidewalls that extend from a base and define a slot, wherein the slot is configured to receive the insert such that an edge of the insert extends beyond the frame and the sidewalls are configured to shrink around a portion of the insert.

2. The instrument of claim 1, wherein the frame is made of a metal powder and a binding agent binding the metal powder.

3. The instrument of claim 1 or claim 2, wherein the insert includes a cutting edge configured to cut through bone tissue.

4. The instrument of any one of claims 1-3, further comprising:
an additional frame extending from the base; and
an additional insert, wherein the additional frame is configured to receive the additional insert.

5. The instrument of claim 4, wherein the insert includes a first tip that is part of the edge of the insert and the additional insert includes a second tip, and wherein the frame and the additional frame are adjacent to each other and oriented such that the first and the second tips point in the same direction and are spaced an equal distance from the base when the insert and the additional insert are inserted into the frame and the additional frame, respectively.

6. The instrument of claim 1, wherein a width of the frame decreases as the frame extends away from the base.

7. The instrument of claim 1, wherein the frame further includes a backwall that extends between the sidewalls.

8. The instrument of claim 1, wherein the frame is integral with the base such that the base and the frame are inseparable.

9. The instrument of claim 1, wherein the insert is formed by subtractive manufacturing.

10. The instrument of claim 1, wherein the frame is formed by additive manufacturing.

11. The instrument of claim 1, wherein the insert is made of a single material.

12. The instrument of claim 1, wherein the insert and the frame are made of different materials.

13. The instrument of claim 4, wherein the insert and the additional insert have different shapes.

14. The instrument of claim 1, wherein the sidewalls are configured to hold the insert in a fixed position after the sidewalls are heated treated.
